# EUROPEAN PATENT APPLICATION

(11) **EP 2 228 057 A1**
(43) Date of publication of application: **15.09.2010**
(21) Application number: 09305202.5
(22) Date of filing: 04.03.2009
(51) Int. Cl.: A61K 9/107, A61K 47/44, A61K 47/18, A61K 31/5575, A61P 27/06

(54) **Cationic oil-in-water emulsions containing prostaglandins and uses thereof**

(71) Applicant: Novagali Pharma S.A., 91000 Evry (FR)
(72) Inventor: Lallemand, Frédéric, 94260, Fresnes (FR); Garrigue, Jean-Sébastien, 91370, Verrieres Le Buisson (FR); Philips, Betty, 92160, Antony (FR)
(74) Representative: Icosa

(57) **Abstract**

The present invention relates to a colloidal cationic oil-in-water emulsion comprising:
-a prostaglandin,
-an oil having a iodine value ≤ 2,
-one or more surfactants including at least one quaternary ammonium compound,
-water,

wherein the prostaglandin/total sum of surfactants mass ratio is comprised between 0.5 and 5.

The present invention also pertains to the use of said cationic oil-in-water emulsions for enhancing the stability of said prostaglandins, for the treatment of ocular hypertension and/or glaucoma, for promoting growth of eyelashes and/or for treating eyelash hypotrichosis.

## Description

The present invention pertains to cationic oil-in-water emulsions containing prostaglandins and their use in order to enhance the stability of said prostaglandins.
The present invention also concerns the use of said cationic oil-in-water emulsions for the topical administration of prostaglandins and in particular for the treatment of ocular hypertension and/or glaucoma, for promoting growth of eyelashes and/or for treating eyelash hypotrichosis.
In the present invention, the term "prostaglandin" is indifferently used for prostaglandins, their derivatives, precursors, prodrugs or analogues.

Glaucoma is a disease characterized by optic nerve damage and visual field defect and is often associated with an increase in the intraocular pressure (IOP). If left untreated, glaucoma can ultimately lead to blindness.
Prostaglandins, such as prostaglandin F_{2α} and its phenyl-substituted analogues, have been shown to effectively reduce the IOP in man and animals. In fact, they have been used in ophthalmic preparations in order to treat glaucoma. For instance, latanoprost is available in the form of a topical eye solution (eyedrops) and sold under the trademark Xalatan^{®},
Indeed, latanoprost is a potent prostaglandin F_{2α} analogue which has been developed for the treatment of glaucoma. Its chemical name is isopropyl - (Z)-7-[(1R,2R,3R,5S)-3,5-dihydroxy-2-[(3R)3-hydroxy-5-phenylpentyl]-cyclopentyl]-5-heptenoate, its molecular formula is C₂₆H₄₀O₅ and its chemical structure is:

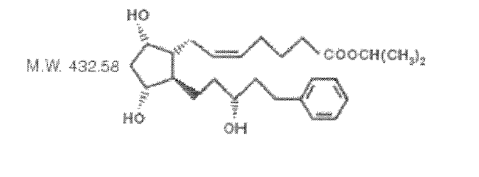

Specifically, latanoprost is a lipophilic prodrug in which the carboxylic acid moiety in the α-chain has been esterified to increase the bioavailability of the active drug into the eye. In addition, latanoprost is absorbed through the cornea where the isopropyl ester prodrug is hydrolyzed to the acid form to become biologically active.
Some ophthalmic prostaglandins, such as bimatoprost, latanoprost or travoprost, have also been described as being capable of promoting eyelash growth. Such prostaglandins could therefore be used for the topical treatment of eyelash hypotrichosis.
The problem generally encountered with prostaglandins is that they may be chemically unstable. In particular, latanoprost is known to be very sensitive towards light and heat. Indeed, these two elements (i.e. light and heat) may have an impact on the stability of latanoprost by provoking its hydrolyzation and/or oxidation. Consequently, stored unopened bottles of Xalatan^{®} should be stored in the dark and under refrigeration at 2-8 °C.
Consequently, there is a need for prostaglandin formulations which show an enhanced chemical stability of the prostaglandin and, in particular, an enhanced stability overtime towards light and heat.
The present invention provides a prostaglandin composition which exhibits an improved stability of the prostaglandin compared to commercial products, while at the same time being non toxic, tolerable for the patient and at least as efficient as the commercially available products.

An object of the present invention is colloidal cationic oil-in-water emulsion comprising:
- a prostaglandin,
- an oil having a iodine value ≤ 2,
- one or more surfactants including at least one quaternary ammonium compound,
- water,
   wherein the prostaglandin/total sum of surfactants mass ratio is comprised between 0.5 and 5.

In particular embodiments of the present invention, the prostaglandin/total sum of surfactants mass ratio in the emulsion is comprised between 0.5 and 4, or between 0.5 and 3, or between 0.5 and 3, or between 0.5 and 2, or between 0.6 and 1.5, or between 0.7 and 1.3, or is around 1.

According to the invention, "colloidal" means that the emulsion comprises colloid particles having an oily core surrounded by an interfacial film dispersed in water with a particle size ≤ 1 µm. Typically, the oily core comprises a prostaglandin and an oil. The prostaglandin being lipophilic, it is thus understandable that it is essentially present in the oily core. Typically, the emulsion may contain other ingredients, such as emollients, preferably glycerol, pH adjusters, such as NaOH, osmotic agents or preservatives.
The cationic emulsion according to the invention is not transparent since the colloidal particles have an average particle size of equal or less than 1 µm, advantageously equal or less than 300 nm, more advantageously in the range of 100 to 250 nm.

In one embodiment, the prostaglandin is a prostaglandin F_{2α}, a derivative, precursor, prodrug or analogue thereof.
In another embodiment, the prostaglandin is an ophthalmic prostaglandin, in particular a prostaglandin which is effective in treating ocular hypertension and/or glaucoma.

In another embodiment, the prostaglandin is an ester prodrug, an amide prodrug of an active prostaglandin, in particular of an active ophthalmic prostaglandin or a mixture thereof. Ester prodrugs include C₁-C₄ alkyl ester prodrugs, such as methyl ester, ethyl ester, isopropyl ester or butyl ester and amide prodrugs include C₁-C₄ alkyl amide prodrugs, such as methyl amide, ethyl amide, isopropyl amide or butyl amide.
According to a particular embodiment, the prostaglandin of the present invention is chosen among latanoprost, isopropyl unoprostone, travoprost, bimatoprost, tafluprost, or mixtures thereof.
Typically, the emulsion according to the present invention comprises latanoprost.
The amount of prostaglandin present in the oily core of the emulsion according to the invention depends on the nature of the prostaglandin and to the intended use. Typically, the amount of prostaglandin relative to the total weight of the emulsion is comprised between 0.001 to 1% w/w, preferably between 0.002 to 0.3% w/w and even more preferably between 0.004 to 0.15% w/w.
In a particular embodiment, the prostaglandin can be combined with other antiglaucoma active ingredients, such as dorsolamide or timolol.

The oil used in the emulsion can be of great importance with respect to the stability of the prostaglandin. According to the present invention, the oil is preferably chosen among saturated oils which are able to limit the degradation of the prostaglandin by oxidation and/or hydrolysis in the emulsion.
According to the invention "saturated oil" is an oil which has an iodine value of less or equal to 2, preferably less than 2, which means that the oil is substantially free of any molecule having a hydrocarbon chain containing double or triple bonds.
The iodine value can be measured for example according to the methods disclosed in the European Pharmacopeia monograph 2.5.4 or US Pharmacopeia monograph 401.
According to a particular embodiment of the present invention, the oil is chosen among oily fatty acids, oily fatty alcohols, fatty acids esters, such as isopropyl myristate and isopropyl palmitate, vegetable oils, animal oils, mineral oils such as petrolatum, liquid paraffin, semi-synthetic oils such as fractionated oils obtained from vegetable oils or mixtures thereof.
According to the invention "semi-synthetic oils" are prepared by chemical synthesis from natural oils.
Particularly, the oil according to the invention is a semi-synthetic oil obtained from fractionated coconut oil, kernel oil or babassu oil. More particularly, the oil is medium chain triglycerides (MCT).

Indeed, according to the European Pharmacopeia, medium-chain triglycerides (MCT) is described as the fixed oil extracted from the hard, dried fraction of the endosperm of *Cocos nucifera* L. by hydrolysis, fractionation of the fatty acids obtained, and re-esterification. MCT consists of a mixture of exclusively short- or medium-chain triglycerides of fatty acids, of which not less than 95% are the saturated fatty acids octanoic (caprylic) acid and decanoic (capric) acid.

Moreover, MCT can also be found in substantial amounts in kernel oil and babassu oil, in addition to some animal products, such as milk-fat, which may contain small amounts (up to 4%) of MCT.
Typically, as the degradation of the prostaglandin by hydrolysis may be pH-dependent, the oil is more preferably chosen among oils which are able to confer to the emulsion a pH which does not substantially cause hydrolysis of the prostaglandin.
In one embodiment, especially when the prostaglandin is an ester prodrug or an amide prodrug of an active prostaglandin (such as latanoprost or bimatoprost) the oil is chosen so as to confer to the emulsion pH values comprised between 4 and 7, particularly between 4.5 and 6.5 and more particularly between 4.5 and 6.
In another embodiment, MCT is used for adjusting the pH of the emulsion between 4 and 7, particularly between 4.5 and 6.5 and more particularly between 4.5 and 6.
In another embodiment, MCT is used in amounts which confer to the emulsion a pH comprised between 4.5 and 6, especially when the emulsion contains latanoprost.
In another embodiment, the pH of the emulsion is preferably comprised between 4 and 7, particularly between 4.5 and 6.5 and more particularly between 4.5 and 6.
In some embodiments, the amount of the oil relative to the total weight of the emulsion is not higher than 7% w/w, preferably between 0.5 and 5% w/w and even more preferably between 1 and 3%w/w.

The emulsion according to the present invention comprises one or more surfactants, including at least one quaternary ammonium compound.

In the emulsion according to the present invention, the quaternary ammonium compound is used as a cationic agent. It is preferably chosen among cetalkonium halide, benzalkonium halide, lauralkonium halide, cetrimide, hexadecyltrimethylammonium halide, tetradecyltrimethylammonium halide, dodecyltrimethylammonium halide, cetrimonium halide, benzethonium halide, behenalkonium halide, cetethyldimonium halide, cetylpyridinium halide, benzododecinium halide, chlorallyl methenamine halide, myristylalkonium halide, stearalkonium halide or mixtures thereof. Halide includes bromide and chloride.

Preferably, the quaternary ammonium compound is chosen among cetalkonium chloride, benzalkonium chloride or mixtures thereof.

The concentration of the quaternary ammonium compound is preferably comprised between 0.001 and 0.1% w/w, more preferably between 0.002 and 0.05% w/w and even more preferably between 0.003 and 0.03% w/w.

The surfactants present in the emulsion of the invention may be chosen among cationic surfactants, anionic surfactants, nonionic surfactants, zwitterionic surfactants, hydrophilic surfactants (with a high HLB), hydrophobic surfactants (with a low HLB) or mixtures thereof. Typically, the choice is made so as to obtain an emulsion which is cationic.
Typically, in addition to quaternary ammonium, the cationic surfactants comprise C10-C24 primary alkylamines, such as oleylamine or stearylamine, tertiary aliphatic amines, cationic lipids, amino alcohols, biguanide salts chosen from chlorhexidine and salts thereof, polyaminopropyl biguanide, phenformin, alkylbiguanide or mixtures thereof, cationic polymers selected from chitosan, 1,2-dioleyl-3-trimethylammonium-propane, 1, 2-dioleoyl-sn-glycero-phosphatidylethanolamine, cationic glycosphingo-lipids or cationic cholesterol derivatives, or mixtures thereof.

Typically, the nonionic surfactants comprise alkyl polyethylene oxide, alkylphenol polyethylene oxide, poloxamers, tyloxapol, alkyl polyglucosides, fatty alcohols, cocamide MEA, cocamide DEA, polyoxyethylene castor oil derivatives, sorbitan esters, polyoxyl stearates, polysorbates or mixtures thereof.
Typically, the anionic surfactants comprise perfluorooctanoate, perfluorooctanesulfonate, alkyl sulphate salts, sodium lauryl ether sulphate, alkyl benzene sulfonate, soaps or fatty acid salts or mixtures thereof.
Typically, the zwitterionic surfactants comprise dodécyl betaine, cocamidopropyl betaine, coco ampho glycinate or mixtures thereof.

Typically, the surfactant according to the invention comprises hydrophilic surfactants (with a high HLB) and /or hydrophobic surfactant (with a low HLB) or mixtures thereof.
In a particular embodiment, the surfactants are chosen among quaternary ammonium compounds, poloxamers, tyloxapol, polysorbates, polyoxyethylene castor oil derivatives, sorbitan esters, polyoxyl stearates or mixtures thereof.

In another embodiment of the invention, the emulsion comprises one or more quaternary ammonium compounds as the only surfactants present in the emulsion. In other words, in this embodiment, the total sum of the surfactants in the emulsion consists of the sum of all quaternary ammonium compound(s).

In a particular embodiment, the emulsion comprises latanoprost, MCT, cetalkonium chloride as the only surfactant and said emulsion has a pH comprised between 5 and 6.

The emulsion according to the invention has a positive zeta potential. This positive zeta potential is preferably higher than 10 mV, more preferably higher or equal to 20 mV.
It has long been recognised that the zeta potential is a very good index of the magnitude of the interaction between colloidal particles and measurements of zeta potential are commonly used to assess the stability of colloidal systems. The zeta potential measured in a particular system is dependent on the chemistry of the surface, and also of the way it interacts with its surrounding environment.
Typically, the emulsions according to the invention are physically stable overtime and keep a positive zeta potential over a period of two years at 25°C. To this end, the emulsions do not contain any substances, or sufficient amount of these substances, susceptible of affecting the zeta potential overtime.
Such substances susceptible of affecting the zeta potential may be phospholipids, or any substances which become negatively charged upon storage. The amount of substances affecting the zeta potential overtime must be such that at any time, the amount of positive charge is above the amount of negative charges.

In a particular embodiment, the emulsion of the invention is free of any buffer.

According to a particular embodiment of the present invention, the emulsion remains stable during autoclaving. According to the present invention, "autoclaving" is defined as sterilization of a product by steam under pressure, by heating said product in an autoclave at high temperatures (e.g. 100 to 200°C, preferably 121°C) during an extended period of time (e.g. 10 to 60 minutes, preferably 10 to 20 minutes) at around 103 kPa (15 psi) above atmospheric pressure. The steam and pressure transfer sufficient heat into organisms to kill them and thus sterilize the product.

Surprisingly, the emulsions of the present invention show a remarkable stability although they contain low amounts of surfactants.

According to the invention, "stability" is defined as the extent to which a product retains, within specified limits and throughout its period of storage and use (i.e., its shelf life), the same properties and characteristics that it possessed at the time of manufacture.

The purpose of stability testing is to provide evidence concerning the quality of a drug substance or a drug product overtime, said product being subjected to a variety of environmental factors such as temperature, humidity and light. The result may be helpful in providing appropriate storage conditions, re-testing periods and shelf lives.

Although conventional stability studies do evaluate those factors which ultimately affect the expiration date of the drugs, these conventional studies are time and cost-consuming. Consequently, in order to predict shelf life of a pharmaceutical product for example, the pharmaceutical industry usually uses "accelerated stability studies" (Stress Test). These accelerated studies help understand the intrinsic stability mechanism of the molecule of interest by establishing degradation pathways and by identifying the likely degradation products. In these types of studies, the products are usually subjected to extreme conditions, such as temperature of about 40°C for approximately 6 months.
In the present invention, the Applicant has developed a "Stress Test" during which the emulsions are subjected to a temperature of 80°C for 14 days.

According to the invention, "good tolerability" means that the ratio "therapeutic benefit" to "ocular discomfort" is acceptable by the patient, and preferably similar to a placebo or NaCl solution 0.9%. It is generally accepted that in order to show good ocular tolerability the cationic agent content within the formulation should not exceed 0.1%, preferably not exceed 0.05% and even more preferably should not exceed 0.03%.

Another object of the present invention is a process for manufacturing the emulsion previously described. The emulsions of the invention are therefore prepared according to the following steps:
- preparation of the oily phase by mixing the prostaglandin (e.g. latanoprost) with the saturated oil (e.g. MCT) and possibly the quaternary ammonium when it is not soluble in the aqueous phase,
- preparation of the aqueous phase by mixing the water-soluble ingredients (e.g. glycerol,) with purified water;
- incorporating the oily phase to the aqueous phase;
- rapidly heating the coarse emulsion obtained, preferably at 75°C;
- decreasing the emulsion droplet size by any suitable means known to the man skilled in the art, for example by shear mixing;
- cooling down the emulsion preferably to about 20°C using an ice bath;
- homogenizing the cooled emulsion;
- optionally, adjusting the pH to a physiological pH, by using for example NaOH or HCl;
- optionally sterilizing the emulsion by autoclaving.

An additional advantage of the emulsions of the invention is that they are stable during the autoclaving, in other words, the prostaglandin is not degraded and the zeta potential remains positive.
An object of the present invention is the cationic oil-in-water emulsion according to the invention for use in a method of treatment.
The emulsion according to the present invention is preferably intended to be applied topically, e.g. to the skin, to the surface of the eye or to hairs, such as eyelashes.

An object of the present invention is the cationic oil-in-water emulsion according to the invention for use in a method for treating ocular hypertension and/or for treating glaucoma.
An object of the present invention is the cationic oil-in-water emulsion according to the invention for use in a method for promoting growth of eyelashes or treating eyelash hypotrichosis.
An object of the present invention is an ophthalmic formulation comprising the cationic oil-in-water emulsion according to the invention, optionally in combination with an ophthalmologically acceptable carrier. It may be in the form of eye drops, eye ointment, or ophthalmic gel.
An object of the present invention is the use of the cationic oil-in-water emulsion according to the invention in order to enhance the chemical stability of prostaglandins.
An object of the present invention is a delivery device comprising the cationic oil-in-water emulsion according to the invention.
Typically the delivery device according to the invention is selected from the group comprising lenses, ocular patch, implant, insert.

Other features and advantages of the invention will emerge upon reading the following non limiting examples.

### EXAMPLES

### 1. Preparation of a cationic oil-in-water emulsion.

The cationic oil-in-water emulsion according to the present invention is prepared by the following steps:
- preparation of the oily phase by mixing at 50°C the prostaglandin (latanoprost) with the saturated oil (MCT) and CKC;
- preparation of the aqueous phase by mixing at 50°C glycerol and purified water;
- incorporating the oily phase to the aqueous phase;
- rapidly heating the coarse emulsion obtained at 75°C;
- decreasing the emulsion droplet size by any suitable means known to the man skilled in the art, for example by shear mixing 5 min at 16 000 rpm (Polytron PT6100; Kinematica, Switzerland)
- cooling down the emulsion to about 20°C using an ice bath;
- homogenizing during 20 min at 15 000 psi the cooled emulsion (Emulsiflex C3, Avestin, Canada)
- sterilizing the emulsion by autoclaving.

The composition of the emulsion is given in table 1.

**Table 1**

| | Ingredients | Supplier | Theorethical composition (% w/w) |
|---|---|---|---|
| Oily phase | MCT (Medium Chain Triglycerides) | Sasol GmbH, Germany | 1.000 |
| | Latanoprost | | 0.005 |
| | CKC | Dishman, India | 0.005 |
| Aqueous phase | Glycerol | Merck, Germany | 2.400 |
| | Water (up to 100) | | 96.590 |
| | NaOH 1M | Merck, Germany | qs pH 7 |
| | Total | | 100% |

After sterilisation, the pH of the emulsion is 4.9.

### 2. Stability Test & Comparative Test

The stability of the emulsion of example 1 was evaluated under accelerated conditions "Stress Test" (at 80°C during 14 days), while a comparative analysis was conducted between the anionic emulsion (invention) and Xalatan^{®} under the same "Stress Test" conditions. Prostaglandin content was analysed in both tests by an HPLC-UV method. The results are given in table 2 (stability test) and table 3 (comparative test).

**Table 2**

| Emulsion of example 1 | Aspect | Zeta potential (mV) | Osmolality (mOsm/kg) | pH | Droplet size (nm) | Latanoprost (% w/w) |
|---|---|---|---|---|---|---|
| T= 0 days | White milky homogeneous emulsion Tyndall effect | 55.2 | 280 | 4.9 | 188 | 0.00511 |
| T= 7 days | White milky homogeneous emulsion Tyndall effect | 44.5 | 277 | 4.86 | 208 | - |
| T= 14 days | White milky homogeneous emulsion Tyndall effect | 42.5 | 281 | 4.88 | 221 | 0.00510 |

**Table 3**

| | Latanoprost (% w/w) | | pH | | Zeta potential (mV) | |
|---|---|---|---|---|---|---|
| | T0 | T14 days | T0 | T14 days | T0 | T14 days |
| Emulsion of example 1 | 0.00511 | 0.00492 | 4.9 | 4.88 | 55.2 | 42.5 |
| Xalatan® | 0.00510 | 0.00248 | 6.74 | 6.71 | NA | NA |

| | | | | | | |
|---|---|---|---|---|---|---|
| NA: not applicable | | | | | | |

At T0, the concentrations in prostaglandins for the emulsion (invention) and for Xalatan^{®} are close to 0.005%. However, after subjecting both emulsions to the "Stress Test" (14 days at 80°C), it can be observed that the concentration of prostaglandins remains the same for the emulsion (invention), while it has decreased by more than half in the case of Xalatan^{®}.

## Claims

1. A colloidal cationic oil-in-water emulsion comprising:
- a prostaglandin,
- an oil having a iodine value ≤ 2,
- one or more surfactants including at least one quaternary ammonium compound,
- water,
wherein the prostaglandin/total sum of surfactants mass ratio is comprised between 0.5 and 5.

2. The colloidal cationic oil-in-water emulsion according to claim 1, **characterized in that** the prostaglandin/total sum of surfactants mass ratio is comprised between 0.5 and **4,** preferably between 0.5 and 3, more preferably between 0.5 and 2, even more preferably between 0.6 and 1.5, even more preferably between 0.7 and 1.3, even more preferably around 1.

3. The colloidal cationic oil-in-water emulsion according to claims **1 or 2, characterized in that** the prostaglandin is chosen among an ester prodrug, an amide prodrug of an active prostaglandin or mixtures thereof.

4. The colloidal cationic oil-in-water emulsion according to claim **3, characterized in that** the prostaglandin is chosen among latanoprost, isopropyl unoprostone, travoprost, bimatoprost, tafluprost, or mixtures thereof.

5. The colloidal cationic oil-in-water emulsion according to anyone of claims **1 to 4,** wherein the quaternary ammonium compound is chosen among cetalkonium halide, benzalkonium halide, lauralkonium halide, cetrimide, hexadecyltrimethylammonium halide, tetradecyltrimethylammonium halide, dodecyltrimethylammonium halide, cetrimonium halide, benzethonium halide, behenalkonium halide, cetethyldimonium halide, cetylpyridinium halide, benzododecinium halide, chlorallyl methenamine halide, myristylalkonium halide, stearalkonium halide or mixtures thereof.

6. The colloidal cationic oil-in-water emulsion according to claim **5,** wherein the quaternary ammonium compound is chosen among cetalkonium chloride, benzalkonium chloride or mixtures thereof.

7. The colloidal cationic oil-in-water emulsion according to any one of claims **1 to 6, characterized in that** the oil is chosen among oily fatty acids, oily fatty alcohols, fatty acids esters, such as isopropyl myristate and isopropyl palmitate, vegetable oils, animal oils, mineral oils such as petrolatum, liquid paraffin, semi-synthetic oils such as fractionated oils obtained from vegetable oils or mixtures thereof.

8. The colloidal cationic oil-in-water emulsion according to claim **7, characterized in that** the oil is fractionated coconut oil, kernel oil or babassu oil.

9. The colloidal cationic oil-in-water emulsion according to claim **8, characterized in that** the fractionated oil is medium chain triglycerides (MCT).

10. The colloidal cationic oil-in-water emulsion according to anyone of claims **1 to 9,** wherein the surfactants are chosen among quaternary ammonium compounds, poloxamers, tyloxapol, polysorbates, polyoxyethylene castor oil derivatives, sorbitan esters, polyoxyl stearates or mixtures thereof.

11. The colloidal cationic oil-in-water emulsion according to anyone of claims **1 to 7,** wherein the amount of prostaglandin relative to the total weight of the emulsion is comprised between 0.001 to 1% w/w, preferably between 0.002 to 0.3% w/w and even more preferably between 0.004 to 0.15% w/w.

12. The colloidal cationic oil-in-water emulsion according to anyone of claims **1 to 11,** wherein the amount of the quaternary ammonium compound relative to the total composition is comprised between 0.001 and 0.1% w/w, preferably between 0.002 and 0.05% w/w and even more preferably between 0.003 and 0.03% w/w.

13. The colloidal cationic oil-in-water emulsion according to anyone of claims **1 to 12,** wherein the amount of the oil relative to the total weight of the emulsion is not higher than 7% w/w, preferably between 0.5 and 5% w/w and even more preferably between 1 and 3%w/w.

14. The colloidal cationic oil-in-water emulsion according to anyone of claims **1 to 13,** said emulsion having a positive zeta potential, preferably higher than 10 mV, more preferably higher or equal to 20 mV.

15. The colloidal cationic oil-in-water emulsion according to anyone of claims **1 to 14,** wherein said emulsion is free of any buffer.

16. The colloidal cationic oil-in-water emulsion according to anyone of claims **1 to 15,** wherein said emulsion has a pH comprised between 4 and 7.

17. The colloidal cationic oil-in-water emulsion according to anyone of claims **1 to 16,** wherein said emulsion remains stable during autoclaving.

18. The colloidal cationic oil-in-water emulsion according to any one of claims **1 to 17,** for use in a method of treatment.

19. The colloidal cationic oil-in-water emulsion according to any one of claims **1 to 18,** for use in a method for treating ocular hypertension and/or for treating glaucoma.

20. The colloidal cationic oil-in-water emulsion according to any one of claims **1 to 18,** for use in a method for promoting growth of eyelashes or for treating eyelash hypotrichosis.

21. Ophthalmic formulation comprising the colloidal cationic oil-in-water emulsion according to any one of claims **1 to 20,** optionally in combination with an ophthalmologically acceptable carrier.

22. Use of the colloidal cationic oil-in-water emulsion according to any one of claims **1 to 17,** to enhance the chemical stability of prostaglandins.

23. Delivery device comprising the colloidal cationic oil-in-water emulsion according to any one of claims **1 to 17.**
